(19) Europäisches Patentamt
European Patent Office
Office européen des brevets

(11) Publication number: 0 335 393 A1

(12) EUROPEAN PATENT APPLICATION

(21) Application number: 89105623.6

(22) Date of filing: 30.03.89

(51) Int. Cl.4: C07C 93/04

(30) Priority: 31.03.88 US 176222

(43) Date of publication of application: 04.10.89 Bulletin 89/40

(84) Designated Contracting States: AT BE CH DE ES FR GB GR IT LI LU NL SE

(71) Applicant: UNION CARBIDE CORPORATION
39 Old Ridgebury Road
Danbury Connecticut 06817(US)

(72) Inventor: Gerkin, Richard Michael
5300 Kensington Drive
Cross Lane 25313 West Virginia(US)
Inventor: Cowherd, Frank Garnett
5326 Luray Ln.
Cross Lane 25313 West Virginia(US)
Inventor: Schreck, David James
5226 Sun Valley Drive
Cross Lane 25313 West Virginia(US)
Inventor: Kirchner, David Lee
2515 Woodland Avenue
South Charleston 25303 West Virginia(US)

(74) Representative: Wuesthoff, Franz, Dr.-Ing. et al
Patentanwälte Wuesthoff -v.
Pechmann-Behrens-Goetz Schweigerstrasse
2
D-8000 München 90(DE)

(54) **Process for manufacture of N-(polyoxyalkyl)-N-(alkyl)amines.**

(57) A process for the manufacture of N-(polyoxyalkyl)-N-(alkyl)amines is provided by reacting an alcohol with a secondary amine.

EP 0 335 393 A1

# PROCESS FOR MANUFACTURE OF N-(POLYOXYALKYL)-N-(ALKYL)AMINES

## Background of the Invention

### Field of the Invention

The present invention relates to a process for the manufacture of amines, and in particular to N-(polyoxyalkyl)-N-(alkyl)amines, by reacting an alcohol with a secondary amine. These N-(polyoxyalkyl)-N-(alkyl)amines are characterized by a high content of secondary amine groups.

### Prior Art

Amination of alcohols with ammonia under various reaction conditions to produce primary amines is well known. Similarly, primary amines react with alcohols to form secondary amines and secondary amines react with alcohols to form tertiary amines.

Of particular interest regarding the present invention is the latter reaction. Significant prior art exists in this general area.

In U.S. - A - 4,638,336 amines are prepared from an aliphatic alcohol and, as aminating agents ammonia, a primary amine and a secondary amine. When the secondary amine was used, the product was a tertiary amine.

In DE 3,539,266 tertiary amines were prepared from the reaction of secondary amines with alcohols in the presence of a hydrogenation/dehydrogenation catalyst.

In JP 62-51646 alpha, omega ditertiary amines formed from the reaction of an alpha,omega diol and a secondary amine in the presence of hydrogen and copper or cobalt catalyst.

In DE 3,432,015 very high tertiary amines were derived from the reaction of dimethylamine amine and a C12 alcohol. Cooper/tin and other catalysts were discussed.

In U.S. - A - 4,625,063 high purity tertiary amines formed from the reaction of an alcohol and a primary or secondary amine where the catalyst contained copper/nickel and a Group VIII metal.

In JP 59-222,448 alkyldimethylamines were produced from the reaction of dimethylamine and an alcohol.

In U.S. - A - 4,404,404 and 4,409,399 tertiary amines were derived from the reaction of dimethylamine and dodecanol in the presence of copper on nickel oxide catalysts.

In U.S. - A - 4,442,306 tertiary amines were produced from the reaction of alcohol and a secondary amine using a copper formate catalyst.

In JP 62-33138 there is described a special catalyst for controlling the reaction of secondary amines with alkane diols to give mono- and ditertiary amine products.

While not exhaustive, these patents clearly indicate that it is generally expected that secondary amines react with alcohols to form the art-expected tertiary amines. Quite unexpectedly, it has been found that under certain reaction conditions, the reaction between an alcohol and a secondary amine will generate a product in which the secondary amine predominates.

## Object of the Invention

It is a primary object of the present invention to provide N-(polyoxyalkyl)-N-(alkyl) amines wherein the content of secondary amine groups exceeds the content of primary and tertiary amine groups.

It is another object of the present invention to provide a novel route to such amines via the reaction of alcohols with secondary amines.

## Summary of the Invention

The present invention provides a novel process for manufacturing N-(polyoxyalkyl)-N-(alkyl) amines. The process is characterized by reacting an alcohol, such as a monol or a polyol with a secondary amine in the presence of an appropriate catalyst, such as nickel, under specific reaction conditions.

## Detailed Description of the Invention

In accordance with the present invention there is provided a process for manufacturing N-(polyoxyalkyl)-N-(alkyl)amines of the general formula:

$R\,[H]_{(hz)}\,[\,(P)_p\,(S)_s\,(T)_t\,]_{z-(hz)}$

wherein R is an initiator radical based on a compound containing Zerewitinoff active hydrogen atoms. Such compounds are capable of initiating polymerization with alkylene oxides when used with a suitable catalyst (e.g., potassium hydroxide, zinc hexacyanocobaltate).

Examples of such compounds include: monofunctional compounds such as methanol, butanol, phenol, nonylphenol, lauryl alcohol, 2-methoxyethanol; difunctional compounds such as ethylene glycol, propylene glycol, water, 1,4-butanediol, diethylene glycol; trifunctional compounds such as trimethylolpropane, glycerine; and other polyfunctional compounds such as pentaerythritol, sorbitol, ammonia, ethylene diamine, 1,3-diaminopropane, 1,6-hexanediamine, isomers of phenylenediamine and toluenediamine, 4,4′-diphenylmethane diamine and its isomers, diethanolamine, ethanolamine, dimethylethanolamine N-methylethanolamine, triethanolamine, triisopropanolamine, ethylmercaptan, thiophenol and propylene disulfide.

Additional examples of compounds suitable for initiating polymerization of alkylene oxides are the various oligomeric polyols known in the art. These include the poly-(1,4-butylene oxide)polyethers and the hydroxyl and amine terminated poly-(butadienes). When polyols (or other oligomers) are used for initiating polymerization of the alkylene oxides, their molecular weights can range from 400 to 3000. When the conventional initiators such as described above (i.e., glycerine, water, etc.) are used, their molecular weight can range from 18 (for water) up to 400. Preferably R contains from 2 to 6 carbon atoms and most preferably 3 to 6 carbon atoms.

The alkylene oxides and monomers that find utility in the present invention are those well known in the art. These include propylene oxide, ethylene oxide, the alpha olefin oxides such as 1,2-epoxybutane and 1,2-epoxyoctadecane, oxetane, and tetrahydrofuran.

"H" denotes the group represented by the formula:

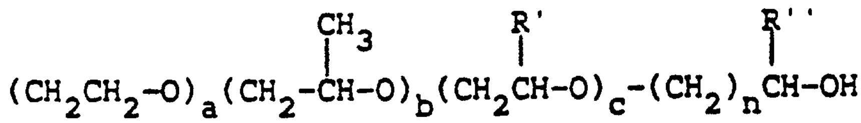

"P" denotes the group represented by the formula:

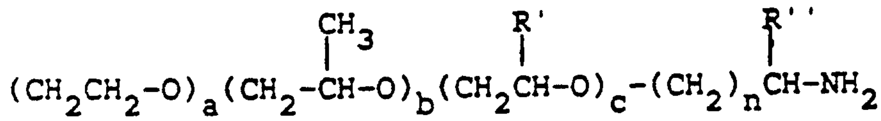

"S" denotes the group represented by the formula:

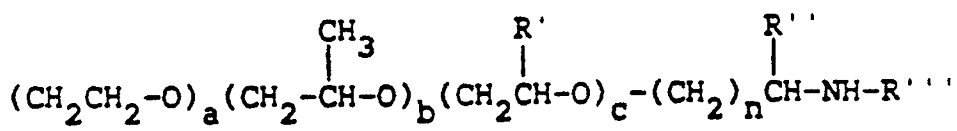

"T" denotes the group represented by the formula:

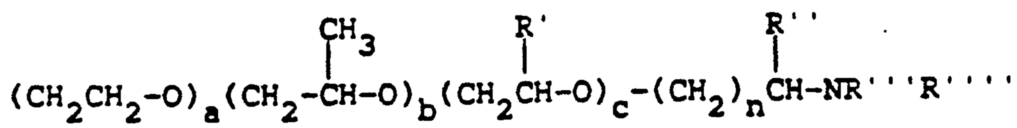

wherein:

The letter "a" defines the ethylene oxide content of the N-(polyoxyalkyl)-N-(alkyl)amine and can range from a value of zero to 175. The preferred range for "a" is 0 to 90. When b or c is not equal to zero, the most preferred range for "a" is 0 to 50.

The letter "b" defines the propylene oxide content of the N-(polyoxyalkyl)-N-(alkyl)amine and can also range from a value of zero to 175. Preferably, "b" should range from 20 to 115 and most preferably from 25 to 98.

The letter "c" defines the alpha olefin oxide

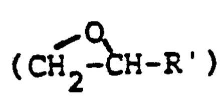

content of the N-(polyoxyalkyl)-N-(alkyl)amine and can range from 0 to 30. Preferably, "c" can range from zero to 15 and most preferably, from 0 to 2.

The letter "n" equals from 1 to 3, preferably 1.

Two aspects of a, b and c are important and must be noted. The first is that the sum of a+b+c must always be greater than or equal to 2 when n equals 1. Second, a, b and c indicate ethylene oxide, propylene oxide and alpha olefin oxide which can be incorporated into the product backbone in any sequence, i.e., blocks or random sequence, in any configuration.

$R'$ is an alkyl group containing from 2 carbon atoms to 18 carbon atoms depending on the alpha olefin oxide used in preparation of the amine. While $R'$ can contain up to 18 carbon atoms, two carbon atoms are most preferred.

$R''$ is hydrogen or an alkyl group containing up to eighteen carbon atoms. It is preferred that $R''$ is hydrogen or an alkyl group containing up to 2 carbons, and most preferably a methyl group

$R'''$ and $R''''$ are independently an alkyl or aryl group containing from 2 to 12 carbon atoms, preferably an alkyl group containing from 2 to 6 carbon atoms, and most preferably an isopropyl group.

The letter "h" indicates the relative hydroxyl content remaining after amination and is from 0 to 0.7, with 0 to 0.3 preferred and 0 to 0.15 most preferred. As noted, "h" is related to the percent amination, i.e. 30 percent amination would result in a hydroxyl content of 70 percent and thus "h" would equal 0.7. Values are obtained by taking the total amine number as measured in milliequivalents per gram, and dividing by the initial hydroxyl number (meg/g) and substracting that quotient from 1.0.

The letter "p" indicates the relative primary amine content to total amine content formed during amination and is from 0 to less than 0.5, preferably 0 to 0.4, most preferably 0 to 0.25.

The letter "s" indicates the relative secondary amine content to total amine content formed during amination and is from 0.5 to 1.0, preferably from 0.7 to 1.0.

The letter "t" indicates the relative tertiary amine content to total amine content formed during amination and is from 0 to 0.15, preferably from 0 to 0.05. The sum of p, s and t must equal 1.0.

The letter "z" is an integer derived from the number of Zerewitinoff active hydrogens on the initiator. The letter "z" is preferably 1 to 6, and most preferably 3 to 6.

The N-(polyoxyalkyl)-N-(alkyl)amines are prepared by direct, catalytic amination of an appropriate alcohol with a secondary amine:

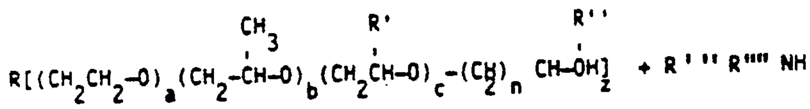

Amines used in the amination are limited to secondary mines. $R'''$ and $R''''$ are as defined above. Representative examples include but are not limited to: diethylamine, di-n- propylamine, di-n-butylamine, diisobutylamine, dicyclohexylamine, diisopropylamine, and diphenylamine. These secondary amines are commercially available from a variety of sources or can be prepared from any of several well known techniques. Generally, secondary amines are prepared by reacting an alcohol with a primary amine or the proper stoichiometric amount of an alcohol with ammonia. Accordingly, the present invention can also be practiced by charging the proper secondary amine reactants in place of the secondary amine itself and allowing the secondary amine to form in situ.

Another method for preparing the secondary amine is described in U.S. - A - 4,286,074, where a primary amine terminated polyether is allowed to react with acetone, with the resulting ketimine being hydrogenated to the product. Although this method is only exemplified for about 1000 equivalent weight materials, it is technically applicable to other molecular weights and functionalities. However, this method is somewhat limited due to the availability of the primary amine terminated polyether.

It should be noted that although the reaction of a primary amine with an alcohol (polyol) is a known approach, nonetheless, U.S. - A -4,686,242 teaches that this approach actually produces an amine terminated polyether where the amine groups are predominantly primary amines.

The alcohols, and especially the monols and polyols used in the present invention, are well known in the art and commercially available from a variety of sources.

The reaction is carried out in a batch autoclave at elevated temperature, generally between 175° C to 250° C and preferably 190° C to 240° C. The reaction pressure will range from 17.25 - 138 bar (250 to 2000 psi), preferably 34.5 - 86.25 bar (500 to 1250 psi). The reaction is run in the presence of hydrogen. Under these conditions the hydroxyl-containing polymer remains in the liquid phase. The stoichiometry on a amine to hydroxyl equivalent basis will range from 2:1 to 20:1, preferably 5:1 to 10:1. The reaction will generally occur in 4 to 24 hours. The catalyst is a nickel, copper or cobalt catalyst, most preferably nickel, either unsupported or on a support. When the catalyst is supported, it is preferred that the metal content of the catalyst be at least 25%, with 50% or more metal content most preferred. The catalyst loading is generally on the order of 1 to 5 weight percent based on total charge.

In addition to the batch process described above, the amination can be carried out using a liquid phase continuous amination process. In this process, a pelletized or extruded form of the nickel, copper or cobalt catalyst, optionally on a support, is charged to a high pressure tubular reactor. Most preferably, a nickel catalyst is employed. The reactor is heated to 175° C to 250° C, preferably 190° C to 240° C and a mixture of amine and polyol (2:1 to 20:1, preferably 5:1 to 10:1 on an equivalents basis) is pumped through the reactor at a flow rate ranging from 0.5 to 5.0 g feed/g catalyst/h . Hydrogen is added to the feed stream at a

minimum rate of 1 standard $cm^3$/min. Reactor pressure is controlled by a back pressure regulator to 17.25-138 bar (250-2000 psi), preferably 34.5-86.25 bar (500-1200 psi). The products isolated from the continuous process are similar to those isolated from the batch process.

The N-(polyoxyalkyl)-N-(alkyl)amines of the present invention find utility in the preparation of polyureas and polyurethane-urea products.

The following specific examples illustrate certain aspects of the present invention. All parts and percentages are by weight unless otherwise specified.

## EXAMPLES

Definitions

Polyol #1 = A polyoxyalkylene oxide triol produced from propylene oxide and glycerine polymerized at 105° C. The final product has an hydroxyl number of 28 (mg KOH/g).

Polyol #2 = A polyoxyalkylene oxide diol produced from propylene oxide and propylene glycol. The final product has an hydroxyl number of 37.5 (mg KOH/g).

Polyol #3 = A polyoxyalkylene oxide hexol produced from propylene oxide and ethylene oxide and sorbitol. The final product as an hydroxyl number of 28 (mg KOH/g). The ethylene oxide content of the final product is 10% by weight, present as an internal block.

Polyol #4 = A polyoxyalkylene oxide triol produced from propylene oxide and glycerine. The final product has an hydroxyl number of 650 (mg KOH/g).

Polyol #5 = A polyoxyalkylene oxide triol produced from propylene oxide, ethylene oxide and glycerine. The final product has an hydroxyl number of 34 mg KOHg. The ethylene oxide is present at a level of 15% as a cap.

Polyol #6 = A polytetramethyleneglycol diol of about 2000 molecular weight.

Polyol #7 = a polyoxyalkylene oxide diol produced from ethylene oxide with a molecular weight of about 400.

Catalyst #1 = A nickel catalyst sold commercially by Harshaw/Filtrol Partnership as Nickel 5136P. 65% nickel on silica-alumina.

Catalyst #2 = A nickel catalyst sold commercially by Harshaw/Filtrol Partnership as Ni-3288E.

Catalyst #3 = A nickel catalyst sold commercially by Harshaw/Filtrol partnership as Ni-3266E. 50% Nickel extrudate.

Catalyst #4 = A copper catalyst sold commercially by Harshaw/Filtrol partnership as Cu-203T. Copper chromite.

Catalyst #5 = A copper catalyst sold commercially by Calsicate as E-408TU. 82% copper oxide on aluminum oxide.

Catalyst #6 = A cobalt catalyst sold commercially by Harshaw/Filtrol partnership as Co-164T 1/8. 25% Cobalt on a proprietary support.

Example 1. Preparation of a 6000 molecular weight N-(polyoxyalkyl)-N-(alkyl)amine with high secondary amine content.

Polyol #1 (800g), diethylamine (294.75g) and Catalyst #1 (28g) were charged to a 2 liter autoclave. This was pressurized to 200 psig and vented five times with hydrogen. After pressurizing to 13.8 bar (200 psig), the reactor was closed and heated to 190° C for 19 hours. After cooling, the catalyst was removed by filtration and the excess amine taken off by vacuum stripping (2 h at 125° C and 10 mbar -8 mm Hg-). The isolated product had the following analysis: Total amine #0.418 meq/g; conversion 85.3%; secondary amine 80.6%*; Primary amine 19.4%*⁻ and tertiary amine #0.035 meq/g.

(*Percentage of total reactive amine. (Total amine - tertiary amine).)

Comparative Example A - Preparation of 6000 molecular weight N-(polyoxyalkyl)-N-(alkyl)amine with low secondary amine content.

Polyol #1 (800g), isopropylamine (223g) and Catalyst #1 (28g) were charged to a 2 liter reactor and the amination carried out as described in Example #1. Following the same workup procedure, the product had the following analysis; Total amine # 0.422 meq/g, conversion 86%; secondary amine 32.4%; primary amine 67.6% and tertiary amine #0.00054 meq/g.

Comparative Examples B - G -

Polyol #1, isopropylamine and Catalyst #1 at the levels described in Comparative Example A were allowed to react under the conditions described below to form N-(polyoxyalkyl)-N-(alkyl)amines with comparatively low secondary amine content (Table I):

Table I

| Comparative Examples Amination with Primary Amine (Isopropylamine) | | | | | | | | |
|---|---|---|---|---|---|---|---|---|
| Comp. Ex. | Reactor | Time | Temp. °C | Total Amine | Conv.,% | 2° % | 1° % | Tertiary, meq/g |
| B | 2 liter | 4 h | 190 | 0.387 | 79 | 26.1 | 73.9 | 0.0041 |
| C | 2 liter | 19 | 190 | 0.362 | 74 | 27.5 | 72.5 | 0.0056 |
| D | 2 liter | 19 | 90 (.5hr) 190 | 0.422 | 86 | 32.5 | 67.5 | 0.00054 |
| E | 2 liter | 19 | 190 | 0.43 | 88 | 35.6 | 64.4 | 0.00046 |
| F | 3.78 liter | 24 | 190 | 0.459 | 94 | 20 | 80 | - |
| G | 3.78 liter | 19 | 190 | 0.465 | 95 | 24.3 | 75.7 | - |

While there is some scatter, the runs at 190 °C give product containing from 20 to about 36% secondary amine and up to 50% has been observed.

Examples 2-7. (Table III)

Preparation of 6,000 molecular weight N-(polyoxyalkyl)-N-(alkyl)amine with high secondary amine content using representative secondary amines as aminating agent and Polyol #1. The conditions and reactor outlined in Example #1 were used to prepare these products. For reference, the amine/polyol equivalent ratio was 10/1.

Table II

| Amination with secondary Amines | | | | | | |
|---|---|---|---|---|---|---|
| Example | Secondary Amine | Amine meq/g | Conversion, % | 2°,% | 1°,% | 3°,% meq/g |
| 2 | di-n-propyl | 0.376 | 76.7 | 74.3 | 25.7 | 0.019 |
| 3 | diisobutyl | 0.386 | 78.7 | 96.0 | 4.0 | 0.065 |
| 4 | di-n-butyl | 0.45 | 91.8 | 84.6 | 15.4 | 0.046 |
| 5 | diisopropyl | 0.474 | 96.7 | 73.0 | 28.0 | 0.0074 |
| 6 | diphenyl | 0.464 | 95.0 | 93.6 | 6.4 | 0.014 |
| 7 | dicyclohexyl | 0.412 | 87.6 | 100 | - | 0.003 |

Example 8.

Preparation 3000 molecular weight, difunctional N-(polyoxyalkyl)-N-(alkyl)amine containing high secondary amine content. Polyol #2 (2000g) diisopropylamine (1357g) and Catalyst #1 (70g) were charged to a 7.6 l (2 gallon) autoclave. The reactor was pressurized three times with hydrogen and vented. The fourth

time the reactor was pressurized to 13.8 bar (200 psi) with hydrogen and sealed. The mixture was heated for 19 hours at 190 °C. After cooling, filtering and vacuum stripping, isolated product had the following analysis: Total amine 0.59 meq/g; conversion 91.7%; secondary amine 86%; primary amine 14%.

Example 9.

Preparation of low molecular weight trifunctional N-(polyoxyalkyl)-N-(alkyl)amine containing high secondary amine content. Polyol #4 (600g) diisopropylamine (3517.6g) and Catalyst #1 (21g) were charged to a 7.6 l (2 gallon) autoclave. The reaction was pressurized with hydrogen to remove air and then pressurized to 13.8 bar (200 psi) with hydrogen and sealed. The mixture was then heated for 19 hours at 190 °C. After cooling, filtering and vacuum stripping, the isolated product had the following analysis:
Total amine 5.62 meq/g; conversion 71.4%; secondary amine 95% primary amine 5%.

Example 10.

Preparation of a 2000 equivalent weight (about 12,000 molecular weight) hexafunctional N-(polyoxyalkyl)-N-(alkyl)amine containing high secondary amine content. Polyol #3 (3000g) diisopropylamine (1528.9g) and Catalyst #1 (105 g) were charged to a 7.6 (2 gallon) autoclave. The reactor was pressurized with hydrogen and vented to remove air and then pressurized to 13.8 bar (200 psi) with hydrogen and sealed. The mixture was then heated for 19 hours at 190 °C. After cooling, filtration and vacuum stripping, the isolated product had the following analysis: Total amine 0.406 meq/g; conversion 86%; secondary amine 75% primary amine 25%.

Example 11.

Preparation of 6000 molecular weight N-(polyoxyalkyl)-N-(alkyl)amine using a mixture of secondary and primary amines. The procedure used was that of Example #1. The diethylamine was replaced with diisopropylamine (308 g) and isopropylamine (44 g), (80/20 mole % respectively, used at a 10:1 equivalent ratio over the polyol) and the reaction was run for 20.5 hours. The isolated product had the following analysis: Total amine 0.457 meq/g; conversion 97.2%; secondary amine 90%; primary amine 10% and tertiary amine 0.017 meq/g.

Example 12.

Preparation of 6000 molecular weight N-(polyoxyalkyl)-N-(alkyl)amine using a mixture of ammonia and isopropyl alcohol (1:2 molar ratio). Polyol #1 (1404.1 g), isopropyl alcohol (424.6 g), ammonia (58.8g) and Catalyst #1 (49.1 g) were charged to a 1 gallon reactor. The reactor was purged with hydrogen to remove air and pressurized to 13.8 bar (200 psi) with hydrogen. The system was heated to 190 C and held at that temperature for 19 hours. Filtration of the catalyst and removal of the excess volatile materials gave a product with the following analysis: Total amine 0.25 meq/g; conversion 50%; secondary amine 64% and primary amine 36%.

Example 13.

Preparation of 6000MW N-(polyoxyalkyl)-N-(alkyl) amine with high secondary amine content via the continuous process. Catalyst #3, 284.5g was charged to a vertical six foot long one inch OD tube (high pressure tubing) equipped controlled heaters and a hydrogen inlet. The catalyst was activated at 150 °C with hydrogen. A mixture of Polyol #1 (66.4%) and diisopropylamine (33.6%) was prepared for subsequent feed to the tubular reactor. Feed was initiated and the following conditions established: Pressure 69 bar (1000 psi) feed rate 627g/h ; Temperature 210 °C; hydrogen flow 22 standard $cm^3$/min. A sample was collected after equilibration of the conditions. After removal of the excess amine by vacuum stripping, the product had the following analysis: Total amine 0.404 meq/g; conversion 82%; secondary amine content 73%; primary amine content 27%.

Example 14 -

Another run was carried out as in Example 13 at 210° C and 293 g/h and gave the following product: Total amine 0.422 meq/g; conversion 86%; secondary amine 58%; primary amine 42%.

Example 15:

Preparation of a 5000MW N-(polyoxyalkyl)-N-(alkyl)amine with high secondary amine content from an ethylene oxide capped polyol. The procedure used was that of Example #1. Polyol #5 (800g), diisopropylamine (489g) and Catalyst #1 (28g) were allowed to react under the indicated conditions. The isolated product had the following analysis: Total amine 0.525 meq/g; conversion 87.5%; primary amine content 0.116 meq/g; tertiary amine content 0.037 meq/g and secondary amine (by difference) 0 .372 meq/g or 70 .8%.

Example 16 =

Preparation of a 6000 molecular weight N-(polyoxyalkyl)-N-(alkyl)amine using a secondary amine with Catalyst #5. The procedure used was that of Example #1. Polyol #1 (1620.5 g), diisopropylamine (640.4 g), and Catalyst #5 (42.6 g -tablets were ground to powder under an inert atmosphere) were charged to a 3.78 l reactor. The reactor was purged with hydrogen to remove air and pressurized to 13.8 bar (200 psi) with hydrogen. The system was heated to 190° C and held at temperature for about 23 hours. Filtration of the catalyst and removal of the excess volatile materials gave a product with the following analysis: Total amine 0.187 meq/g; conversion 50%; secondary amine 93% and primary amine 7%.

Example 17 =

Preparation of a 6000 molecular weight N-(polyoxyalkyl)-N-(alkyl)amine using a secondary amine with a Catalyst #4. The procedure used was that of Example # 1. Polyol #1 (1258.2 g), diisopropylamine (639.6 g), and Catalyst #4 (42.6 g -tablets were ground to powder under an inert atmosphere) were charged to a 3.78 l reactor. The reactor was purged with hydrogen to remove air and pressurized to 13.8 bar (200 psi) with hydrogen. The system was heated to 190° C and held at temperature for about 21 hours. Filtration of the catalyst and removal of the excess volatile materials gave a product with the following analysis: Total amine 0.134 meq/g; conversion 27%; secondary amine 95% and primary amine 5%.

Example 18 =

Preparation of a 6000 molecular weight N-(polyoxyalkyl)-N-(alkyl)amine using a secondary amine with Catalyst #6. The procedure used was that of Example # 1. Polyol #1 (1260.3 g), diisopropylamine (643.5 g), and Catalyst #6 (46.6 g -tablets were ground to powder under an inert atmosphere) were charged to a 3.78 l reactor. The reactor was purged with hydrogen to remove air and pressurized to 13.8 bar (200 psi) with hydrogen. The system was heated to 190° C and held at temperature for about 22 hours. Filtration of the catalyst and removal of the excess volatile materials gave a product with the following analysis: Total amine 0.252 meq/g; conversion 51%; secondary amine 92.4% and primary amine 7.6%. Tertiary amine was 0.003 meq/gram.

Example 19 =

Preparation of about a 2000 molecular weight N-(polytetramethyleneglycol)-N-(alkyl)amine using a secondary amine with Catalyst #1. The procedure used was that of Example #1. Polyol #6 (515.9 g), diisopropylamine (523.8 g), and Catalyst #1 (23.3 g) were charged to a 2 liter reactor. The reactor was purged with hydrogen to remove air and pressurized to 13.8 bar (200 psi) with hydrogen. The system was heated to 190° C and held at temperature for about 21.5 hours. Filtration of the catalyst and removal of the excess volatile materials gave a product with the following analysis: Total amine 0.768 meq/g; conversion

80%; secondary amine 94% and primary amine 6%.

Example 20

Preparation of about a 400 molecular weight N-(polyoxyethyleneoxide)-N-(alkyl)amine using a secondary amine with a nickel catalyst. The procedure used was that of Example #1. Polyol #7 (538.0 g), diisopropylamine (1357.8 g), and Catalyst #I (42.6 g) were charged to a 3.78 l reactor. The reactor was purged with hydrogen to remove air and pressurized to 13.8 bar (200 psi) with hydrogen. The system was heated to 190 C and held at temperature for about 23.5 hours. Filtration fo the catalyst and removal of the excess volatile materials gave a product with the following analysis: Total amine 3.78 meq/g; conversion 91%; secondary amine 92% and primary amine 8%.

## Claims

1. A process for preparing N-(polyoxyalkyl)-N-(alkyl)amines of the general formula:
$R\,[H]_{(hz)}\,[\,(P)_p\,(S)_s\,(T)_t\,]_{z-(hz)}$
wherein :R is an initiator radical based on a compound containing Zerewitinoff active hydrogen atoms;

H is the group represented by the formula:

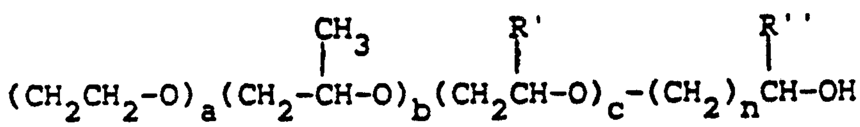 ;

P is the group represented by the formula:

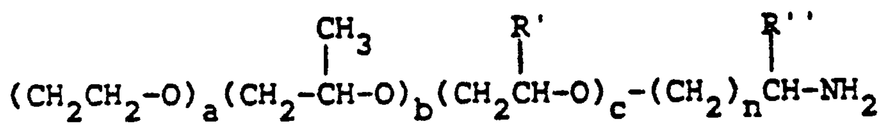 ;

S is the group represented by the formula:

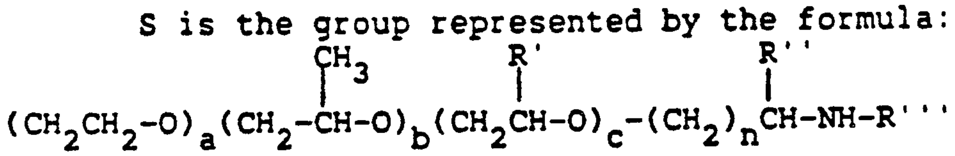 ;

T is the group represented by the formula:

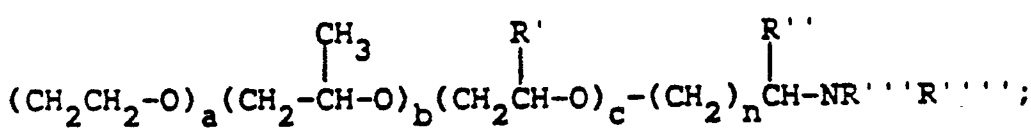

a is 0 to 175;
b is 0 to 175;
c is 0 to 30;
n is 1 to 3;
the sum a + b + c ≧ 2, when n equals 1;
R′ is an alkyl group containing from 2 carbon atoms to 18 carbon atoms;
R″ is hydrogen or an alkyl group containing up to 18 carbon atoms;
R‴ and R⁗ are independently an alkyl or aryl group containing from 2 to 12 carbon atoms;
h is 0 to 0.7;
p is 0 to < 0.5;
s is 0.5 to 1.0;
t is to 0.15; and

z is a integer derived from the number of Zerewitinoff active hydrogens on the initiator. which process comprises the reaction of an alcohol of the general formula

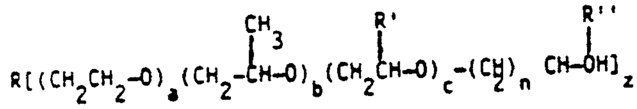 + R'''R''''NH

with a secondary amine of the general formula
R''' R'''' NH
in a nickel, copper cobalt catalyzed reaction the reaction occurring at a temperature of 175°C to 250°C and a pressure of 17.25-138 bar with the ratio of amine to hydroxyl equivalent from 20:1 to 10:1.

2. The process of claim 1 wherein the secondary amine is selected from diethylamine, di-n-propylamine, di-n-butylamine, diisobutylamine, dicyclohexylamine, diisopropylamine and diphenylamine.

3. The process of claim 1 or 2 wherein the alcohol is a monol or polyol wherein
R contains from 2 to 6 carbon atoms,
a is 0 to 150,
b is 20 to 115,
c is 0 to 15,
n is 1 to 3
R is an alkyl group containing 2 carbon atoms, and
z is 1 to 6.

4. The process of claim 3 wherein z is 3 to 6.

5. The process of claims 1 to 4 wherein the process is carried out in a batch mode or in a continuous mode at a temperature between 175° to 250°C, and a pressure between 17.25 - 138 bar with a nickel catalyst.

6. The process of claim 5 whrein the temperature is between 190° to 240°C and the pressure is between 34.5 -86.25 bar.

European Patent Office

# EUROPEAN SEARCH REPORT

Application number

EP 89105623.6

**DOCUMENTS CONSIDERED TO BE RELEVANT**

| Category | Citation of document with indication, where appropriate, of relevant passages | Relevant to claim | CLASSIFICATION OF THE APPLICATION (Int. Cl.4) |
|---|---|---|---|
| A | EP - A2 - 0 180 455 (ATLANTIC RICHFIELD) * Claims 1,4,5,7,8,9; tables * | 1,2 | C 07 C 93/04 |
| A | US - A - 4 487 967 (STOGRYN et al.) * Claims 1,5,9,10; example 1 * | 1,5,6 | |
| A | DE - A1 - 3 641 666 (KAO CORP.) * Claims 1,4; example 1 * | 1 | |
| A | GB - A - 1 589 167 (BASF) * Claims 1,8,11; example 4 * | 1 | |

TECHNICAL FIELDS SEARCHED (Int. Cl.4)

C 07 C 93/00
C 07 C 85/00

The present search report has been drawn up for all claims

| Place of search | Date of completion of the search | Examiner |
|---|---|---|
| VIENNA | 04-07-1989 | KÖRBER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document